# NEUE EUROPÄISCHE PATENTSCHRIFT

(11) **EP 3 113 756 B2**
(45) Veröffentlichungstag und Bekanntmachung des Hinweises auf die Entscheidung über den Einspruch: **04.10.2023**
(45) Hinweis auf die Patenterteilung: 13.01.2021
(21) Anmeldenummer: 15702801.0
(22) Anmeldetag: 06.02.2015
(51) Int. Cl.: A61K 8/44, A61Q 19/10, A61K 8/92

(54) **ABRASIVE HAUTREINIGUNGSMITTEL I**
ABRASIVE SKIN CLEANING PRODUCTS I
PRODUITS DE NETTOYAGE DE LA PEAU ABRASIFS I

(30) Priorität: 05.03.2014 DE 102014204056
(43) Veröffentlichungstag der Anmeldung: 11.01.2017
(73) Patentinhaber: Beiersdorf AG, 20253 Hamburg (DE)
(72) Erfinder: KÖNIG, Sylvia, 21635 Jork (DE); OTTO, Sebastian, 20253 Hamburg (DE); ARGEMBEAUX, Horst, 21465 Wentorf (DE)
(74) Vertreter: Beiersdorf AG
(86) Internationale Anmeldenummer: PCT/EP2015/052502
(87) Internationale Veröffentlichungsnummer: WO 2015/132040

(56) Entgegenhaltungen:
- EP-A1- 0 566 339
- EP-A1- 2 431 451
- WO-A1-99/22712
- WO-A1-2004/035720
- WO-A1-2006/120003
- WO-A1-2012/047957
- WO-A1-2015/028418
- WO-A2-02/46342
- WO-A2-2007/102957
- DE-A1- 4 039 244
- DE-A1- 4 403 710

## Beschreibung

Die Hautreinigung ist von je her ein Bedürfnis des Menschen. Die Methoden und Mittel zur Hautreinigung haben sich im Laufe der Zeit verändert. Heute besteht die Hautreinigung häufig darin sich mit seifenhaltigen oder seifenartigen Produkten zu waschen, mit Duschzubereitungen zu duschen und mit Badeseifen oder Badezubereitungen zu baden. In der Regel reichen diese Verfahren aus um die Haut zu reinigen und zu erfrischen.

Es gibt jedoch Situationen, in denen eine intensivere Reinigung erforderlich ist. Dies ist zum Beispiel der Fall bei stark verschmutzten Hautoberflächen. Eine solch starke Verschmutzung ist bei manchen Berufsgruppen anzutreffen, z.B. bei Automechanikern. Ölhaltige Verschmutzungen lassen sich oftmals mit gewöhnlichen festen oder flüssigen Seifen oder Handreinigungsmitteln nicht entfernen.

Auch bei einer tiefergehenden Hautreinigung, bei der abgestorbene Hornzellen der oberen Hautschicht abgetragen werden sollen um ein gleichmäßigeres Hautbild zu erhalten, reichen normale Hautreinigungsmittel nicht aus.

In beiden Fällen ist es wünschenswert, dass neben der Reinigung durch Wasser und Tenside eine zusätzliche mechanische Reinigung der Haut erfolgt. Hierzu werden tensidhaltige Reinigungsmittel mit Partikeln versetzt. Diese Partikel sind in der Regel Polyethylenpartikel oder pulverisierte Pflanzenbestandteile wie Obstkerne oder Nussschalen. Die Polyethylenpartikel werden jedoch nur sehr schlecht bis gar nicht in den Klärwerken biologisch abgebaut. In der Folge akkumulieren sie in den Flüssen und Meeren. Sie stellen damit eine Gefahr für die Fischpopulationen dar.

Auch die Partikel aus pulverisierten Pflanzenbestandteilen weisen Nachteile auf, denn diese Partikel sind anfällig für Verkeimungen. Um diese Verkeimungen zu verhindern werden die Partikel aus pulverisierten Pflanzenbestandteilen häufig mit Gamma-Bestrahlung behandelt, einem Verfahren, dem die Verbraucher jedoch kritisch gegenüber stehen. Darüber hinaus haben die Partikel aus pulverisierten Pflanzenbestandteilen meist eine braune Farbe. Dies hat Auswirkungen auf das kosmetische Endprodukt, das von den Verbrauchern aufgrund von dunkleren Einfärbungen daher als "unkosmetisch" eingestuft wird.

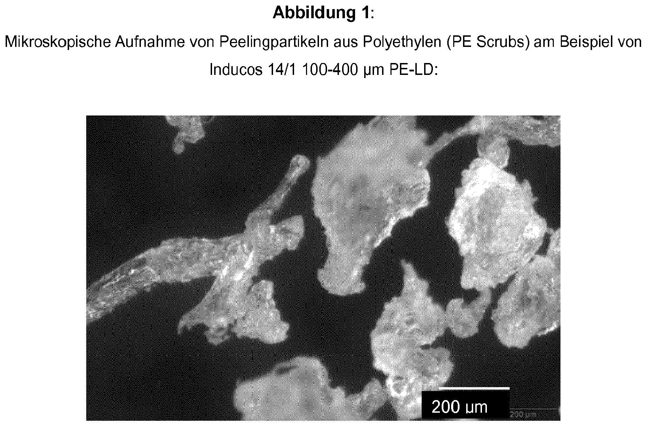

EP 2338962 A1 und EP 2338964 A2 offenbaren abrasive Partikel, die in Reinigungszubereitungen einsetzbar sind. Diese Partikel sind durch unregelmäßige Oberflächen mit teilweise herausragenden Bereichen (Figur 1, EP 2338962 A2). Ein weiterer Nachteil liegt in der Tatsache begründet, dass die Partikel, die als Abrasiva eingesetzt werden, eine recht breite Verteilung in der Partikelgröße aufweisen. Zur Illustration der Partikelgrößenverteilung und der Partikeloberfläche wie sie im Stand der Technik anzutreffen ist, wird in Abbildung 1 eine Aufnahme von Polyethylenpartikeln gezeigt. Es wird deutlich, dass die Oberflächen nicht gleichmäßig rund erscheinen. Darüber hinaus sind Partikel in verschiedenen Größen mit sehr verschiedenen Formen zu erkennen. Das Erscheinungsbild ist inhomogen.

Wenn Partikel mit einer Größe > 600 µm Durchmesser, insbesondere aber > 850 µm Durchmesser in tensidhaltige Reinigungszubereitungen eingearbeitet werden, ist die Sensorik in derartigen Reinigungszubereitungen beeinträchtigt. Beim Auftrag dieser Zubereitungen auf die Haut entsteht ein kratziger Eindruck. Dies ist beim Verbraucher unerwünscht. Darüber hinaus kann die Einarbeitung derartig großer Partikel zu Mikroverletzungen der Haut führen. Diese Mikroverletzungen sind umso problematischer, wenn bei unbeabsichtigtem Kontakt, Augen oder Schleimhäute betroffen sind. Die Mikroverletzungen können besonders ausgeprägt sein, wenn beim Verteilen einer Zubereitung, die derartig große Partikel enthält, die Partikel mehrmals auf der Hautoberfläche hin und her gerieben werden.

Hautreinigungsmittel sind in der Regel stark wasserhaltig und bedürfen einer sorgfältigen Konservierung um eine mikrobiologischen Kontamination zu vermeiden. Zur Konservierung sind ausschließlich Konservierungsmittel zugelassen, die in einer Positivliste der Kosmetikverordnung (Deutschland) oder in der Verordnung (EG) Nr. 1223/2009 der EU über kosmetische Mittel genannt werden
Es gibt wissenschaftliche Studien, die eine Verbindung von bestimmten Konservierungsmitteln und dem Auftreten von Allergien aufzuzeigen versuchen. Ebenso gibt es Untersuchungen, die einen möglichen Einfluss bestimmter Konservierungsmittel auf das Hormonsystem nahelegen. Wie oben ausgeführt sind alle Konservierungsmittel, die in der Kosmetik eingesetzt werden dürfen, in der oben genannten Positivliste aufgeführt. Diese Konservierungsmittel sind als unbedenklich im Einsatz in Kosmetika eingestuft.

Dennoch ist es aus Gründen, die die Galenik oder die Technik bestimmte Rezepturen stabil zu formulieren, betreffen, angezeigt bestimmte Konservierungsmittelklassen bevorzugt zu verwenden.

Es ist also Aufgabe der vorliegenden Erfindung Reinigungszubereitungen zur Verfügung zu stellen, die Peelingkörper mit einer guten biologischen Abbaubarkeit aufweisen und darüber hinaus zu einer Verbesserung in der Sensorik der Reinigungszubereitungen führen. Weiterhin ist es Aufgabe der vorliegenden Erfindung Reinigungszubereitungen enthaltend Peelingkörper zur Verfügung zu stellen, die ein oder mehrere Konservierungsmittel enthalten, die die Verkeimung der Zubereitungen verhindern, d.h. für antimikrobiell stabile Zubereitungen sorgen.

Weiterhin ist es die Aufgabe, dass Reinigungszubereitungen enthaltend Tenside und Peelingkörper sich durch eine gute Schaumleistung auszeichnen.

Darüber hinaus sollen diese Zubereitungen ein Fließverhalten aufweisen, dass eine gute Entnahme aus dem jeweiligen Packmittel ermöglicht.

Überraschenderweise werden alle vorgenannten Aufgaben gelöst durch Hautreinigungszubereitungen, enthaltend Tenside und Peelingpartikel, wobei die Peelingpartikel kugelförmig sind und aus mindestens einer wachsartigen Substanz bestehen und wobei die Zubereitungen frei sind von Formaldehyd-abspaltenden Konservierungsmitteln, 2-Bromo-2-Nitropropane-1,3-Diol und Butylparaben,
- wobei die Tenside ausgewählt werden aus der Gruppe der anionischen, amphoteren und nichtionischen Tenside und wobei die Tenside mit einem Gehalt von 5 bis 18 Gew.-%, bevorzugt 10 bis 15 Gew.-%, insbesondere bevorzugt 10 bis 13 Gew.-%, bezogen auf das Gesamtgewicht der Zubereitung, vorliegen,
- die anionischen Tenside Alkali- und/oder Ammoniumsalze der Alkylethersulfate sind, wobei die Alkylethersulfate Alkylreste mit einer Kettenlänge von 8 bis 16 Kohlenstoffatomen, insbesondere Alkylreste mit einer Kettenlänge von 10 bis 14 Kohlenstoffatomen und ganz insbesondere Alkylreste mit einer Kettenlänge von 11 bis 13 Kohlenstoffatomen, aufweisen,
- wobei die wachsartigen Substanzen ausgewählt werden aus der Gruppe Carnaubawachs, Candellila Wachs, Sonnenblumenwachs, hydriertes Rhizinusöl, Reiswachs, hydriertes Sonneblumenöl, hydriertes Sojaöl, Paraffin, wobei Hartparaffin, Mikrokristallines Wachs Vaseline umfasst sind, und Stearyl Stearat und
- wobei der überwiegende Anteil der Peelingpartikel eine Partikelgröße ≤ 850 µm Durchmesser aufweist, wobei bevorzugt ist, dass der Anteil der Peelingpartikel mit einem Durchmesser von 150 bis 450 µm 60 bis 75 Gew.-% beträgt und wobei
- die erfindungsgemäßen Zubereitungen wenigstens ein Konservierungsmittel aus der Gruppe Benzoesäure, ein Salz der Benzoesäure, Salicylsäure und ein Salz der Salicylsäure, enthalten oder wobei
   die erfindungsgemäßen Zubereitungen wenigstens ein Konservierungsmittel aus der Gruppe Methylparaben und Ethylparaben enthalten.

Es ist bevorzugt, dass die Peelingpartikel nur aus einer wachsartigen Substanz bestehen, besonders bevorzugt aus hydriertem Rizinusöl.

Es ist ebenso bevorzugt, dass die Peelingpartikel aus mehreren wachsartigen Substanzen bestehen.

Es ist ebenso bevorzugt, dass die Peelingpartikel Mischungen von Peelingpartikeln sind, wobei die Partikel nur aus einer wachsartigen Substanz oder aus mehreren wachsartigen Substanzen bestehen können. Es ist besonders bevorzugt, wenn diese Mischungen von Peelingpartikeln Peelingpartikel enthalten, die aus hydriertem Rizinusöl bestehen.

Im Sinne der Erfindung bestehen die wachsartigen Partikel nur aus wachsartigen Substanzen oder im Wesentlichen aus wachsartigen Substanzen.

Im Wesentlichen bedeutet, dass die Peelingpartikel mindestens zu 90 % aus der oder den jeweiligen Substanze(n) bestehen.

Wachse im Sinne der Erfindung sind Substanzen, die über 40°C ohne Zersetzung schmelzen. Oberhalb des Schmelzpunktes sind sie nicht fadenziehend und ziemlich niedrig viskos. Nach der Herkunft teilt man Wachse in drei Klassen ein, natürliche Wachse, wie beispielsweise Carnaubawachs und Candelillawachs; chemisch modifizierte Wachse, wie beispielsweise hydrierte Jojobawachse; synthetische Wachse, wie beispielsweise Polyalkylenwachse. Wachsartig bedeutet, dass die eingesetzten Substanzen die oben beschriebenen Eigenschaften aufweisen.

Die Peelingpartikel aus den genannten wachsartigen Substanzen weisen eine gute biologische Abbaubarkeit auf.

In den erfindungsgemäßen Zubereitungen können weitere Peelingpartikel vorhanden sein. Diese weiteren Peelingpartikel haben eine andere chemische Zusammensetzung als die kugelförmigen Peelingpartikel, die aus wachsartigen Substanzen bestehen. Bevorzugt sind hierbei Peelingpartikel aus natürlichen oder naturbasierten Substanzen. Besonders bevorzugt sind Partikel ausgewählt aus der Gruppe Partikel aus Hydrated Silica, Polymilchsäure und mikrokristalliner Cellulose. Die Partikel aus Polymilchsäure können nur aus Polymilchsäure oder im Wesentlichen aus Polymilchsäure bestehen. Das Gewichtsverhältnis der Peelingpartikel bestehend aus mindestens einer wachsartige Substanz zu den Peelingpartikeln bestehend aus mindestens einer Substanz ausgewählt aus der Gruppe Hydrated Silica, Polymilchsäure und mikrokristalliner Cellulose, beträgt 10:1 bis 1:10, bevorzugt 3:2 bis 2:3.

Der Gesamtgehalt an Peelingpartikeln in den Reinigungszubereitungen beträgt 0,05 bis 15 Gew.-%, bevorzugt 0,075 bis 10 Gew.-%, insbesondere bevorzugt 0,1 bis 7 Gew.-%, bezogen auf das Gesamtgewicht der Zubereitung.

Erfindungsgemäß sind Zubereitungen mit Peelingpartikeln in einer definierten Größe. Erfindungsgemäß sind Peelingpartikel, bei denen der überwiegende Anteil der Peelingpartikel eine Partikelgröße ≤ 850 µm Durchmesser aufweist. Bevorzugt sind Peelingpartikel mit einem Anteil von 60 bis 75 Gew.-% an Peelingpartikeln, die einen Durchmesser von 150 bis 450 µm aufweisen. Die Größenbestimmung erfolgte gemäß DIN 66165. Derartige Partikel sind beispielsweise zu erwerben als WorléeBeads HCO White 60/100 mesh.

Vorteilhaft sind auch Peelingpartikel, bei denen der überwiegende Anteil eine Partikelgröße ≤ 600µm aufweist, wobei Peelingpartikel bevorzugt sind, die mit einem Anteil ≥ 80 Gew.-% eine Partikelgröße von 250 bis 420 µm aufweisen. Die Größenbestimmung erfolgte gemäß DIN 66165. Derartige Partikel sind beispielsweise zu erwerben als WorléeBeads HCO White 20/60 mesh.

Suspendierte Partikel, die eine Peelingfunktion in der Zubereitung erfüllen, werden als Peelingpartikel oder Peelingkörper bezeichnet. Die Begriffe Peelingpartikel, Peelingmittel oder Peelingkörper werden in dieser Offenbarung synonym verwendet.

Neben den erfindungsgemäß bevorzugt verwendeten Peelingpartikeln können in den erfindungsgemäßen Zubereitungen weitere Peelingmittel enthalten sein. Auch möglich, wenngleich nicht erwünscht, ist der Einsatz von Peelingmitteln, wie beispielsweise Tonerde, Sand, zerstoßene oder zermahlene Kerne von Walnussschalen, Aprikosen-, Pfirsich- und/oder Mandelkernen. Ebenso möglich, wenngleich auch nicht erwünscht ist der Einsatz vollsynthetischer Peelingmittel.

Die erfindungsgemäßen Hautreinigungsmittel, die Peelingpartikel und Tenside enthalten, sind stark wasserhaltig und damit keimanfällig. Derartige Hautreinigungsmittel bedürfen einer ausgewogenen Konservierung. Konservierungsmittel können, in verschiedene Klassen eingeteilt werden.

Zur Gruppe der halogenorganischen Konservierungsmittel wird beispielsweise 2-Bromo-2-Nitropropane-1,3-Diol gerechnet, ebenso 5-Chlor-2-(2,4-dichlorphenoxy)-phenol (Triclosan), lodopropynyl Butylcarbamate, Methylchloroisothiazolinone, Methyldibromo Glutaronitrile (MDGN), Chloracetamide, Dichlorphenyl-Imidazoldioxolan und andere. Die erfindungsgemäßen Zubereitungen sind frei von 2-Bromo-2-Nitropropane-1,3-Diol. Bevorzugt ist es, dass die erfindungsgemäßen Zubereitungen frei sind von halogenorganischen Verbindungen.

Formaldehydabspalter sind Substanzen, die Formaldehyd abspalten und als Konservierungsmittel zum Einsatz kommen können. Es gibt eine ganze Reihe von derartigen Substanzen, in der Kosmetik werden häufiger Substanzen der Gruppe Diazolidinyl Urea (Germall II^{®}), Imidazolidinyl Urea (Germall 115^{®}), DMDM Hydantoin, MDM Hydantoin und seltener Quaternium 15 verwendet. Die erfindungsgemäßen Zubereitungen sind frei von Formaldehyd-abspaltenden Substanzen.

Butylparaben wird zur Gruppe der Paraben-haltigen Konservierungsmittel gerechnet. Die allgemeine Strukturformel der Parabene ist im Folgenden dargestellt:

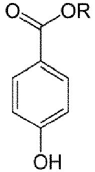

R bedeutet Alkylseitenkette, die aus einer unverzweigten oder verzweigten Kette von 1 bis 5 Kohlenstoffatomen oder aus aromatischen Resten bestehen kann. Bekannte Parabene sind Methyl-, Ethyl-, Propyl- Butylparaben, ebenso wie Isopropyl-, Isobutyl-, Pentyl- und Phenylparaben. Benzylparaben ist in kosmetischen Mitteln als Konservierungsmittel nicht zugelassen. Die erfindungsgemäßen Zubereitungen sind frei von Butylparaben. Es ist bevorzugt, dass die erfindungsgemäßen Zubereitungen frei sind von Parabenen, die eine Alkylseitenkette von mehr als 3 Kohlenstoffatomen aufweisen. Es ist möglich und in bestimmten Fällen vorteilhaft, dass die erfindungsgemäßen Zubereitungen frei sind von Parabenen, ausgewählt aus der Gruppe Methyl-, Ethyl-, Propyl-, Isopropyl-, Isobutyl-, Pentyl- und Phenylparaben.

In Sinne der Erfindung bedeutet frei von, dass maximal 0,001 Gew.-%, von den entsprechenden Substanzen enthalten sind. Diese Gehalte sind lediglich bedingt durch die Zugabe von weiteren Bestandteilen in die Zubereitungen, die beispielsweise diese Substanzen, insbesondere Konservierungsstoffe enthalten können.

Es ist erfindungsgemäß, wenn die erfindungsgemäßen Zubereitungen als Konservierungsmittel Benzoesäure und/oder ein Salz der Benzoesäure enthalten. Ebenso erfindungsgemäß ist es, wenn als Konservierungsmittel Gleichfalls erfindungsgemäß ist es, wenn als Konservierungsmittel Ethylparaben und/oder Methylparaben eingesetzt wird.

Die erfindungsgemäß einsetzbaren Konservierungsstoffe können auch ausgewählt werden aus der Gruppe Phenoxyethanol, Benzylalkohol und Benzylsalicylat. Darüber hinaus gibt es Wirkstoffe mit antimikrobieller Sekundärwirkung, die die Wirkung der Konservierungsmittel ergänzen oder verstärken.

Vorteilhaft ist ebenfalls die Konservierung mit Konservierungsmitteln, die in der Lebensmittelindustrie gebräuchlich sind, wie beispielsweise Sorbinsäure und/oder Sorbate.

Die Konservierungsmittel aus der Gruppe der Parabene werden mit einem Gehalt von 0,02 bis 0,8 Gew.-%, bevorzugt 0,2 bis 0,4 Gew.-%, bezogen auf das Gesamtgewicht der Zubereitung eingesetzt. Die Konservierungsmittel aus der Gruppe der konservierend wirkenden Säuren, wie beispielsweise Benzoesäure und/oder Salicylsäure und/oder ihre Salze werden mit einem Gehalt von 0,04 bis 1,0 Gew.-%, bevorzugt 0,45 bis 0,6 Gew.-%, bezogen auf das Gesamtgewicht der Zubereitung eingesetzt.

Die erfindungsgemäßen Hautreinigungszubereitungen sind dadurch gekennzeichnet, dass sie Tenside enthalten.

Die Tenside werden ausgewählt werden aus der Gruppe der anionischen, amphoteren und nichtionischen Tenside. In den erfindungsgemäßen Zubereitungen ist mindestens ein Tensid aus der angeführten Gruppe enthalten.

Die anionischen Tenside sind Alkali- und/oder Ammoniumsalze der Alkylethersulfate. Besonders bevorzugt sind Alkylethersulfate mit Alkylresten mit einer Kettenlänge von 8 bis 16 Kohlenstoffatomen. Insbesondere bevorzugt sind Alkylethersulfate mit Alkylresten mit einer Kettenlänge von 10 bis 14 Kohlenstoffatomen. Ganz insbesondere bevorzugt sind Alkylethersulfate mit Alkylresten mit einer Kettenlänge von 11 bis 13 Kohlenstoffatomen.

Es ist weiter bevorzugt, dass die amphoteren Tenside Alkyl- und/oder Alkylamidopropylbetaine sind. Besonders bevorzugt sind Alkyl- und/oder Alkylamidopropylbetaine mit Alkylresten mit einer Kettenlänge von 8 bis 16 Kohlenstoffatomen. Insbesondere bevorzugt sind Alkyl- und/oder Alkylamidopropylbetaine mit Alkylresten mit einer Kettenlänge von 10 bis 14 Kohlenstoffatomen. Ganz insbesondere bevorzugt sind Alkyl- und/oder Alkylamidopropylbetaine mit Alkylresten mit einer Kettenlänge von 11 bis 13 Kohlenstoffatomen.

Es ist auch besonders bevorzugt, dass die Alkyl- und/oder Alkylamidopropylbetaine Alkylgruppen aufweisen, die aus der Aufarbeitung von Kokosöl zu Tensiden stammen. Insbesondere bevorzugt ist, dass die Alkyl- und/oder Alkylamidopropylbetaine Cocco-Betaine und/oder Cocoamidopropylbetaine sind.

Es ist weiter bevorzugt, dass nichtionischen Tenside ethoxilierte Glycerinester sind. Besonders bevorzugt sind ethoxilierte Glycerinester mit Alkylresten mit einer Kettenlänge von 8 bis 16 Kohlenstoffatomen. Insbesondere bevorzugt sind ethoxilierte Glycerinester mit Alkylresten mit einer Kettenlänge von 10 bis 14 Kohlenstoffatomen. Ganz insbesondere bevorzugt sind ethoxilierte Glycerinester mit Alkylresten mit einer Kettenlänge von 11 bis 13 Kohlenstoffatomen. Noch weiter bevorzugt ist es, dass die ethoxilierten Glycerinester PEG-7 Glyceryl Cocoate sind.

Erfindungsgemäß beträgt der Gehalt der Tenside in den Hautreinigungszubereitungen 5-18 Gew.-%, bevorzugt 10-15 Gew.-%, insbesondere bevorzugt 10,5 - 13 Gew.-%, bezogen auf das Gesamtgewicht der Zubereitung.

Es können weitere Tenside in den erfindungsgemäßen Zubereitungen zum Einsatz kommen, dies können anionische und/oder amphotere und/oder nichtionische Tenside sein.

Vorteilhaft zu verwendende anionische Tenside sind
Acylaminosäuren (und deren Salze), wie
1. Acylglutamate, beispielsweise Natriumacylglutamat, Natrium Cocoylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
3. Sarcosinate, beispielsweise Myristoyl Sarcosin, TEA-Iauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat.

Carbonsäuren und Derivate, wie
1. Carbonsäuren, beispielsweise Laurinsäure, Aluminiumstearat, Magnesiumalkanolat und Zinkundecylenat, Stearinsäure/-salz, Palmitinsäure/-salz,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-Phosphat, Sulfonsäuren und Salze, wie
1. Acyl-isethionate, z.B. Natrium-/ Ammoniumcocoyl-isethionat, Natrium Lauryl Methylisethionate,
2. Alkylarylsulfonate,
3. Alkylsulfonate, beispielsweise Natriumcocosmonoglyceridsulfat, Natrium C12-14 Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat mit unterschiedlichen Ethoxylierungsgraden und deren Gemische, beispielsweise Natrium-, Ammonium-, Magnesium-, MIPA-, TIPA-Laureth-X-sulfat, Natriummyreth-X-sulfat und Natrium C12-13-Pareth-X-sulfat, mit X = 1-5 Ethoxygruppen.
2. Alkylsulfate, beispielsweise Natrium-, Ammonium- und TEA-Laurylsulfat, Natrium-Ammonium- und TEA-Cocosulfat.

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Dinatrium Cocoamphodiacetat, Dinatrium Cocoamphomonoacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.
3. Betaine, beispielsweise Coco Betaine, Cocoamidopropyl Betaine,
4. Sultaine, beispielsweise Lauryl Hydroxy Sultaine.

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, Laureth-X mit X = 2 bis 10, wobei X Ethoxygruppe bedeutet, ethoxylierte/ propoxylierte Ester, ethoxylierte/ propoxylierte Glycerinester, PEG-200 Hydrogenated Glyceryl Palmat, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Um den erfindungsgemäßen Zubereitungen rückfettende Eigenschaften zu verleihen, können beispielsweise Öle in die erfindungsgemäßen Zubereitungen eingearbeitet werden.

Die Öle können ausgewählt werden aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.
Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* erhältlich ist.

Es ist ferner bevorzugt, die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C12-13-Alkyllactat, Di-C12-13-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C12-15-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Ölkomponenten sind vorteilhaft im Sinne der vorliegenden Erfindung einzusetzen.

Ferner können optional auch unpolare Öle enthalten sein, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen. Diese unpolaren Öle liegen nicht in Partikelform vor.

Ferner können die Zubereitungen optional einen Gehalt an cyclischen oder linearen Silikonölen aufweisen.

Besonders vorteilhaft ist beispielsweise die Verwendung von Coco-Caprylate/Caprate.

Erfindungsgemäße Zubereitungen können ein oder mehrere polymere Strukturanten enthalten. Die polymeren Strukturanten können vorteilhaft gewählt werden aus der Gruppe der Gummen, Polysaccharide, Cellulosederivate, Schichtsilikate, Polyacrylate und/oder anderen Polymeren.

Zu den Gummen zählt man Pflanzen- oder Baumsäfte, die an der Luft erhärten und Harze bilden oder Extrakte aus Wasserpflanzen. Aus dieser Gruppe können beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi ausgewählt werden; besonders vorteilhaft ist Xanthangummi.

Weiterhin vorteilhaft ist die Verwendung von derivatisierten Gummen wie z.B. Hydroxypropyl Guar (Jaguar^{®} HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum^{®}. Diese können als solche oder in modifizierter Form verwendet werden wie z.B. Stearylalkonium Hektorite.

Vorteilhaft sind außerdem Taurate, z.B. Ammonium Acryloyldimethyltaurat / VP Copolymer.

Unter den Polyacrylaten befinden sich z.B. Carbopol Typen der Firma Lubrizol (Carbopol 980, 981, 1382, 5984, 2984, ETD 2001, ETD 2020, ETD 2050, Ultrez-10 oder Pemulen TR1 & TR2), Acrylates Copolymer (Handelsname Carbopol Aqua SF-1) oder Acrylates Crosspolymer-4 (Handelsname: Carbopol Aqua SF-2).

Unter den Polymeren befinden sich z.B. Polyacrylamide (Seppigel 305), Polyvinylalkohole, PVP, PVP / VA Copolymere, Polyglycole.

Erfindungsgemäß besonders bevorzugt ist ein Acrylic Acid/VP Crosspolymer von der Firma ASI für Reinigungszubereitungen enthaltend Kristall-stabilisierende Polyole.

Erfindungsgemäß sind auch Substanzen, die üblicherweise eingesetzt werden, um den pH-Wert der erfindungsgemäßen Zubereitungen einzustellen und stabil zu halten. Vorteilhaft können diese Substanzen ausgewählt werden aus der Gruppe Zitronensäure und Milchsäure und/oder aus der Gruppe Aminomethylpropanol, Natronlauge, Kalilauge und Triethanolamin. Optional können, wenn erforderlich, die in der Kosmetik üblichen Zusatz- und Hilfsstoffe in die Zubereitungen eingearbeitet werden, wie z.B. desodorierend wirkende Substanzen, Antitranspirantien, Insektenrepellentien, Vitamine, Mittel zum Verhindern des Schäumens, Farbstoffe, Pigmente mit färbender Wirkung, Geschmacksstoffe, Vergällungsmittel, Parfüms, weichmachende Substanzen, anfeuchtende und/oder feuchthaltende Substanzen, Antioxidantien, UV-Filtersubstanzen, Sensorikadditive, Filmbildner, Wirkstoffe oder andere übliche Bestandteile einer kosmetischen Formulierung wie Alkohole, Polyole, Schaumstabilisatoren, Elektrolyte, organische Lösungsmittel oder Silikonderivate.

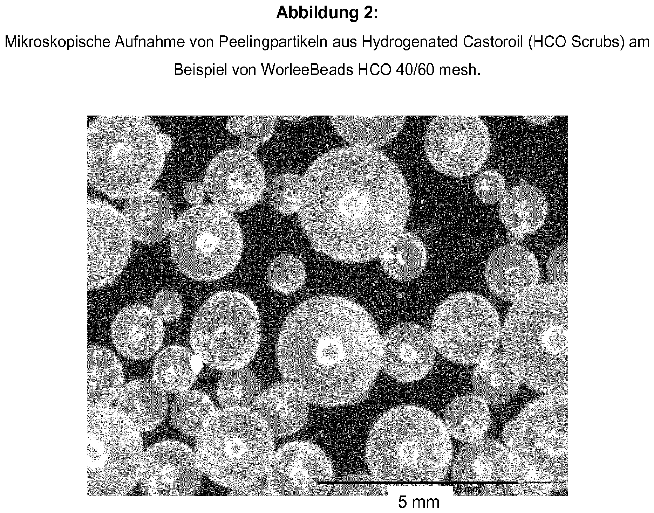

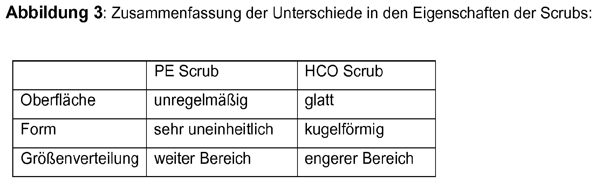

Die Abbildungen 1 und 2 zeigen mikroskopische Aufnahmen von Peelingpartikeln wie sie aus dem Stand der Technik bekannt sind und Peelingpartikeln, die in den erfindungsgemäßen Hautreinigungszubereitungen zum Einsatz kommen. Die Oberfläche der Peelingpartikel aus Polyethylen (PE Scrubs) am Beispiel von Inducos 14/1 100-400 µm PE-LD, die in Abbildung 1 gezeigt werden, weist ein unregelmäßiges Aussehen auf, während die Peelingpartikel aus Hydrogenated Castoroil (HCO Scrubs) am Beispiel von WorleeBeads HCO 40/60 mesh, die in Abbildung 2 dargestellt werden, eine glatte Oberfläche aufweisen. Die Formen der Partikel aus Polyethylen, die in Abbildung 1 dargestellt werden, sind unregelmäßig und uneinheitlich, während die Form der Partikel aus Hydrogenated Castor Oil, die Abbildung 2 dargestellt werden, kugelförmig ist. Die Größenverteilung der Partikel weist ebenfalls Unterschiede auf. In der Partikelpopulation der Polyethylenpartikel (Abbildung 1) sind kleinere Partikel neben größeren Partikeln zu erkennen, während die Größenverteilung der Partikel aus Hydrogenated Castor Oil (Abbildung 2) ein homogeneres Bild zeigt. Zusammenfassend belegen diese Daten, warum die Sensorik der erfindungsgemäßen Hautreinigungszubereitungen deutlich angenehmer ist als bei Zubereitungen des Standes der Technik. Dies ist bedingt durch die Peelingpartikel, die eine kugelige oder weitgehend kugelige Form aufweisen und aus mindestens einer wachsartigen Substanz bestehen.
Die Mengenangaben in der vorliegenden Erfindung beziehen sich auf Aktivgehalte und sind immer Angaben in Gew.-%; es sei denn, es werden ausdrücklich abweichende Angaben gemacht. Die Mengenangaben in Gew.-% in den nachfolgenden Beispielen sind Einsatzkonzentrationen der verwendeten Komponenten oder im Handel erhältlichen Vormischungen.

### Beispiele:

| Duschgele: | | Gew.-% | | | | | |
|---|---|---|---|---|---|---|---|
| **INCI** | **Rohstoff- / Handelsname** | **1** | **2** | **3** | **4** | **5** | **6** |
| PEG-40 Hydrogenated Castor Oil | Sympatens TRH/400, Kolb | 0.40 | 0.40 | 0.40 | 0.40 | 0.40 | 0.40 |
| Parfum | Parfum | 0.80 | 0.80 | 0.80 | 0.80 | 0.80 | 0.80 |
| Aqua + Sodium Hydroxide | Natronlauge 45% | 0.20 | 0.30 | 0.20 | 0.30 | 0.20 | 0.20 |
| Citric Acid | CITRIC ACID MONOHYDRATE | 0.60 | 0.60 | 0.60 | 0.60 | 0.60 | 0.60 |
| Aqua + Styrene/Acrylates Copolymer | Opulyn 301, Dow Chemical | 1.00 | 1.20 | 0.80 | 1.10 | 1.00 | 1.20 |
| Copernica Cerifera Cera | NATUREBEAD B20, Impag | 0.50 | | | | | |
| Candilla Cera | Candelillawachsperlen | | 2.00 | | | | 1.00 |
| Helianthus Annuus Seed Cera | Sonnenblumenwachsperlen | | | 2.00 | | | |
| Oryza Sativa Bran Wax | Natureblue 20RS, Micro Powders | | | | 3.00 | | |
| Hydrogenated Sunflower Seed Oil | Sonnenblumenwachsperlen | | | | | 2.50 | |
| Hydrogenated Soybean Oil | Sojaölperlen | 1.00 | | | | | |
| Paraffin | Paraffinperlen | | 1.00 | | | | |
| Cera Microcristallina | WorléeBeads CMSnow White 40/60 mesh | | | 1.00 | | | |
| Synthetic Wax | WorléeBeads SYN Snow White 40/60 mesh | | | | 1.00 | | |
| Hydrogenated Jojoba Oil | WorléeBeads Jojoba Snow White40/60 mesh | | | | | 1.00 | |
| Stearyl Stearat. | Stearyl Stearat Perlen | | | | | | 3.50 |
| Hydrogenated Castor Oil | Worleé Beads HCO 20/60mesh | 3.50 | 2.00 | 2.00 | 1.00 | 1.50 | 0.50 |
| Lactose + Cellulose + CI 77007 + Hydroxypropyl Methylcellulose | Unispheres UE-507, Induchem | 0.10 | 0.10 | 0.10 | 0.10 | 0.10 | 0.10 |
| Sodium Benzoate | Natrium Benzoat | 0.60 | 0.55 | 0.60 | 0.60 | 0.55 | 0.60 |
| Aqua + Acrylates Copolymer | Carbopol Aqua SF-1 Polymer, Lubrizol Advanced Materials | 4.50 | 5.00 | 4.50 | 5.50 | 4.50 | 5.00 |
| PEG-200 Hydrogenated Glyceryl Palmate + Aqua | Rewoderm LI 520-70, Evonik Industries | 0.20 | 0.10 | 0.20 | 0.10 | 0.20 | 0.20 |
| Aqua | Wasser | 64.60 | 68.95 | 64.80 | 63.50 | 64.65 | 63.90 |
| Sodium Laureth Sulfate + Aqua | Texapon N 70, ca. 70%, BASF Personal Care and Nutrition | 13.00 | 10.00 | 11.00 | 9.00 | 12.00 | 13.00 |
| Aqua + Cocamidopropyl Betaine + Glycerin | Tego-Betain F 50, Evonik Industries | 9.00 | 7.00 | 11.00 | 13.00 | 10.00 | 9.00 |

Duschgele 4 und 5 sind Vergleichsrezepturen

| Gesichtsreinigungsgele: | | Gew .-% | | | | | |
|---|---|---|---|---|---|---|---|
| **INCI** | **Rohstoff- / Handelsname** | **1** | **2** | **3** | **4** | **5** | **6** |
| Parfum | Parfum | 0.10 | 0.10 | 0.10 | 0.10 | 0.10 | 0.10 |
| Glycerin + Aqua | Refined Glycerin 99% | 1.50 | 1.50 | 1.70 | 2.00 | 1.50 | 1.80 |
| Aqua + Sodium Hydroxide | Natronlauge 45% | 0.77 | 0.77 | 0.77 | 0.77 | 0.77 | 0.77 |
| Nylon-11 | Rilsan T Nat BHV Cos, Arkema | 2.50 | 2.90 | 2.90 | 2.70 | 2.90 | 2.80 |
| Copernica Cerifera Cera | NATUREBEAD B20, Impag | 0.50 | | | | | |
| Candilla Cera | Candelillawachsperlen | | 0.20 | | | | 1.00 |
| Helianthus Annuus Seed Cera | Sonnenblumenwachsp erlen | | | 0.50 | | | |
| Oryza Sativa Bran Wax | Natureblue 20RS, Micro Powders | | | | 0.40 | | |
| Hydrogenated Sunflower Seed Oil | Sonnenblumenwachsp erlen | | | | | 0.80 | |
| Hydrogenated Soybean Oil | Sojaölperlen | | 0.80 | | | | |
| Paraffin | Paraffinperlen | | | 0.50 | | | |
| Cera Microcristallina | WorléeBeads CMSnow White 40/60 mesh | | | | 0.60 | | |
| Synthetic Wax | WorléeBeads SYN Snow White 40/60 mesh | | | | | 0.20 | |
| Hydrogenated Jojoba Oil | WorléeBeads Jojoba Snow White40/60 mesh | | | | | | 0.10 |
| Stearyl Stearat. | Stearyl Stearat Perlen | 0.50 | | | | | |
| Hydrogenated Castor Oil | Worleé Beads HCO 20/60mesh | | | | | | |
| Lactose + Cellulose + CI 77007 + Hydroxypropyl Methylcellulose | Unispheres UE-507, Induchem | 0.60 | 0.40 | 0.40 | 0.40 | 0.40 | 0.40 |
| Methylparaben | Solbrol M, Lanxess | 0.40 | 0.30 | 0.30 | 0.35 | 0.30 | 0.35 |
| Phenoxyethanol | Phenoxyethanol P5, Omya Peralta | 0.80 | 0.70 | 0.70 | 0.80 | 0.70 | 0.80 |
| Acrylates/C10-30 Alkyl Acrylate Crosspolymer | Carbopol 1382, Lubrizol Advanced Materials | 1.00 | 0.80 | 0.90 | 0.85 | 0.90 | 0.92 |
| Aqua | Wasser | 89.53 | 89.93 | 89.73 | 89.43 | 89.73 | 89.16 |
| Sodium Laureth Sulfate + Aqua | Texapon N 70, ca. 70%, BASF Personal Care and Nutrition | 1.20 | 1.00 | 0.90 | 1.00 | 1.10 | 1.20 |
| Lauryl Glucoside + Aqua | Plantacare 1200 UP, BASF Personal Care and Nutrition | 0.60 | 0.60 | 0.60 | 0.60 | 0.60 | 0.60 |

Gesichtsreinigungsgele 1 bis 6 sind Vergleichsrezepturen

| Flüssigseife: | | Gew.-% | | | | | |
|---|---|---|---|---|---|---|---|
| **INCI** | **Rohstoff- / Handelsname** | **1** | **2** | **3** | **4** | **5** | **6** |
| PEG-40 Hydrogenated Castor Oil | Sympatens TRH/400, Kolb | 0.60 | 0.70 | 0.50 | 0.60 | 0.70 | 0.70 |
| PEG-7 Glyceryl Cocoate | Cetiol HE, BASF Personal Care and Nutrition | 1.50 | 1.50 | 1.50 | 1.50 | 1.50 | 1.50 |
| Parfum | Parfum | 1.00 | 1.00 | 1.00 | 1.00 | 1.00 | 1.00 |
| Aqua + Sodium Hydroxide | Natronlauge 45% | 0.44 | 0.44 | 0.44 | 0.44 | 0.44 | 0.44 |
| Copernica Cerifera Cera | NATUREBEAD B20, Impag | 0.50 | | | | | |
| Candilla Cera | Candelillawachsperlen | | 2.00 | | | | 1.00 |
| Helianthus Annuus Seed Cera | Sonnenblumenwachsperlen | | | 2.00 | | | |
| Oryza Sativa Bran Wax | Natureblue 20RS, Micro Powders | | | | 3.00 | | |
| Hydrogenated Sunflower Seed Oil | Sonnenblumenwachsperlen | | | | | 2.50 | |
| Hydrogenated Soybean Oil | Sojaölperlen | 1.00 | | | | | |
| Paraffin | Paraffinperlen | | 1.00 | | | | |
| Cera Microcristallina | WorléeBeads CMSnow White 40/60 mesh | | | 1.00 | | | |
| Synthetic Wax | WorléeBeads SYN Snow White 40/60 mesh | | | | 1.00 | | |
| Hydrogenated Jojoba Oil | WorléeBeads Jojoba Snow White40/60 mesh | | | | | 1.00 | |
| Stearyl Stearat. | Stearyl Stearat Perlen | | | | | | 3.50 |
| Hydrogenated Castor Oil | Worleé Beads HCO 20/60mesh | 3.00 | | | | | |
| Lactose + Cellulose + CI 77007 + Hydroxypropyl Methylcellulose | Unispheres UE-507, Induchem | 0.10 | 0.10 | 0.10 | 0.10 | 0.10 | 0.10 |
| Methylparaben | Solbrol M, Lanxess | 0.40 | 0.35 | 0.45 | 0.30 | 0.35 | 0.40 |
| Phenoxyethanol | Phenoxyethanol P5, Omya Peralta | 0.90 | 1.00 | 0.90 | 1.00 | 1.00 | 0.90 |
| Ethylparaben | Ethylparaben, Schütz & Co. | 0.45 | 0.45 | 0.35 | 0.45 | 0.45 | 0.45 |
| Aqua + Acrylates Copolymer | Carbopol Aqua SF-1 Polymer, Lubrizol Advanced Materials | 6.50 | 7.00 | 6.00 | 6.50 | 6.70 | 6.90 |
| Aqua | Wasser | 59.61 | 60.46 | 61.76 | 60.11 | 60.26 | 59.11 |
| Sodium Laureth Sulfate + Aqua | Texapon N 70, ca. 70%, BASF Personal Care and Nutrition | 9.00 | 7.00 | 11.50 | 9.50 | 13.00 | 9.50 |
| Aqua + Cocamidopropyl Betaine + Glycerin | Tego-Betain F 50, Evonik Industries | 13.00 | 15.00 | 10.50 | 12.50 | 9.00 | 12.50 |
| Aqua + Disodium Cocoyl Glutamate + Propylene Glycol | Protelan AGL 95/C, Zschimmer & Schwarz | 2.00 | 2.00 | 2.00 | 2.00 | 2.00 | 2.00 |

Flüssigseifen 4 und 5 sind Vergleichsrezepturen

## Patentansprüche

1. Hautreinigungszubereitungen, enthaltend Tenside und Peelingpartikel, wobei die Peelingpartikel kugelförmig sind und aus mindestens einer wachsartigen Substanz bestehen und wobei die Zubereitungen frei sind von Formaldehyd-abspaltenden Konservierungsmitteln, 2-Bromo-2-Nitropropane-1,3-Diol und Butylparaben,
- wobei die Tenside ausgewählt werden aus der Gruppe der anionischen, amphoteren und nichtionischen Tenside und wobei die Tenside mit einem Gehalt von 5 bis 18 Gew.-%, bevorzugt 10 bis 15 Gew.-%, insbesondere bevorzugt 10 bis 13 Gew.-%, bezogen auf das Gesamtgewicht der Zubereitung, vorliegen,
- die anionischen Tenside Alkali- und/oder Ammoniumsalze der Alkylethersulfate sind, wobei die Alkylethersulfate Alkylreste mit einer Kettenlänge von 8 bis 16 Kohlenstoffatomen, insbesondere Alkylreste mit einer Kettenlänge von 10 bis 14 Kohlenstoffatomen und ganz insbesondere Alkylreste mit einer Kettenlänge von 11 bis 13 Kohlenstoffatomen, aufweisen,
- wobei die wachsartigen Substanzen ausgewählt werden aus der Gruppe Carnaubawachs, Candellila Wachs, Sonnenblumenwachs, hydriertes Rizinusöl, Reiswachs, hydriertes Sonnenblumenöl, hydriertes Sojaöl, Paraffin, wobei Hartparaffin, Mikrokristallines Wachs, Vaseline umfasst sind, und Stearyl Stearat und
- wobei der überwiegende Anteil der Peelingpartikel eine Partikelgröße ≤ 850 µm Durchmesser aufweist, wobei bevorzugt ist, dass der Anteil der Peelingpartikel mit einem Durchmesser von 150 bis 450 µm 60 bis 75 Gew.-% beträgt und wobei
- die erfindungsgemäßen Zubereitungen wenigstens ein Konservierungsmittel aus der Gruppe Benzoesäure, ein Salz der Benzoesäure, Salicylsäure und ein Salz der Salicylsäure, enthalten oder wobei
die erfindungsgemäßen Zubereitungen wenigstens ein Konservierungsmittel aus der Gruppe Methylparaben und Ethylparaben enthalten.

2. Zubereitung nach Anspruch 1, **dadurch gekennzeichnet, dass** die Peelingpartikel nur aus einer wachsartigen Substanz, bevorzugt aus hydriertem Rizinusöl bestehen.

3. Zubereitung nach Anspruch 1, **dadurch gekennzeichnet, dass** ein Peelingpartikel aus mehreren wachsartigen Substanzen besteht.

4. Zubereitung nach Anspruch 2 und/oder 3, **dadurch gekennzeichnet, dass** die Peelingpartikel Mischungen von Peelingpartikeln sind, wobei die Peelingpartikel jeweils nur aus einer wachsartigen Substanz oder aus mehreren wachsartigen Substanzen bestehen.

5. Zubereitung nach wenigstens einem der vorstehenden Ansprüche, **dadurch gekennzeichnet, dass** weitere Peelingpartikeln aus mindestens einer Substanz ausgewählt aus der Gruppe Hydrated Silica, Polymilchsäure und mikrokristalliner Cellulose enthalten sind.

6. Zubereitung nach wenigstens einem der vorstehenden Ansprüche, **dadurch gekennzeichnet, dass** das Gewichtsverhältnis der kugelförmigen Peelingpartikel und bestehend aus mindestens einer wachsartigen Substanz zu den Peelingpartikeln bestehend aus mindestens einer Substanz ausgewählt aus der Gruppe Hydrated Silica, Polymilchsäure und mikrokristalliner Cellulose, 10:1 bis 1:10, bevorzugt 2:3 bis 3:2 beträgt.

7. Zubereitung nach einem der vorstehenden Ansprüche, **dadurch gekennzeichnet, dass** der Gesamtgehalt an Peelingpartikeln 0,05 bis 15 Gew.-%, bevorzugt 0,075 bis 10 Gew.-%, insbesondere bevorzugt 0,1 bis 7 Gew.-%, bezogen auf das Gesamtgewicht der Zubereitung, beträgt.

8. Zubereitung nach wenigstens einem der vorstehenden Ansprüche, **dadurch gekennzeichnet, dass** der überwiegende Anteil der Peelingpartikel eine Partikelgröße ≤ 600 µm Durchmesser, wobei bevorzugt ist, dass der Anteil der Peelingpartikel mit einem Durchmesser von 250 bis 420 µm 80 Gew.-% oder mehr beträgt.

9. Zubereitung nach wenigstens einem der vorstehenden Ansprüche, **dadurch gekennzeichnet, dass** die amphoteren Tenside Alkyl- und/oder Alkylamidopropylbetaine sind, wobei die Alkyl- und/oder Alkylamidopropylbetaine Alkylreste mit einer Kettenlänge von 8 bis 16 Kohlenstoffatomen, insbesondere Alkylreste mit einer Kettenlänge von 10 bis 14 Kohlenstoffatomen und ganz insbesondere Alkylreste mit einer Kettenlänge von 11 bis 13 Kohlenstoffatomen, aufweisen.

10. Zubereitung nach wenigstens einem der vorstehenden Ansprüche, **dadurch gekennzeichnet, dass** die Alkyl- und/oder Alkylamidopropylbetaine Alkylgruppen aufweisen, die aus der Aufarbeitung von Kokosöl zu Tensiden stammen.

11. Zubereitung nach Anspruch 10, **dadurch gekennzeichnet, dass** die Alkyl- und/oder Alkylamidopropylbetaine Cocco-Betaine und/oder Cocoamidopropylbetaine sind.

12. Zubereitung nach wenigstens einem der vorstehenden Ansprüche, **dadurch gekennzeichnet, dass** die nichtionischen Tenside ethoxilierte Glycerinester.

13. Zubereitung nach wenigstens einem der vorstehenden Ansprüche, **dadurch gekennzeichnet, dass** die ethoxilierten Glycerinester Alkylreste mit einer Kettenlänge von 8 bis 16 Kohlenstoffatomen, insbesondere 10 bis 14 Kohlenstoffatomen, ganz insbesondere 11 bis 13 Kohlenstoffatomen aufweisen.

14. Zubereitung nach wenigstens einem der vorstehenden Ansprüche, **dadurch gekennzeichnet, dass** die ethoxilierten Glycerinester PEG-7 Glyceryl Cocoate sind.

15. Zubereitung nach wenigstens einem der vorstehenden Ansprüche, **dadurch gekennzeichnet, dass** die Zubereitungen frei sind von halogenorganischen Konservierungsmitteln.

16. Zubereitung nach wenigstens einem der vorstehenden Ansprüche, **dadurch gekennzeichnet, dass** die Zubereitungen frei sind von Paraben-haltigen Konservierungsmitteln aufweisend eine Alkylseitenkette von mehr als 3 Kohlenstoffatomen.

## Claims

1. Skin cleansing preparations comprising surfactants and exfoliating particles, wherein the exfoliating particles are spherical and consist of at least one waxy substance and wherein the preparations are free from formaldehyde-releasing preservatives, 2-bromo-2-nitropropane-1,3-diol and butylparaben,
- wherein the surfactants are selected from the group of anionic, amphoteric and non-ionic surfactants and wherein the surfactants are present at a content of 5 to 18% by weight, preferably 10 to 15% by weight, particularly preferably 10 to 13% by weight, based on the total weight of the preparation,
- the anionic surfactants are alkali metal and/or ammonium salts of alkyl ether sulfates, wherein the alkyl ether sulfates comprise alkyl radicals having a chain length of 8 to 16 carbon atoms, particularly alkyl radicals having a chain length of 10 to 14 carbon atoms and very particularly alkyl radicals having a chain length of 11 to 13 carbon atoms,
- wherein the waxy substances are selected from the group comprising carnauba wax, candelilla wax, sunflower seed wax, hydrogenated castor oil, rice wax, hydrogenated sunflower seed oil, hydrogenated soybean oil, paraffin, wherein hard paraffin, microcrystalline wax, Vaseline are included, and stearyl stearate and
- wherein the predominant proportion of the exfoliating particles have a particle size diameter of ≤ 850 µm, wherein it is preferred that the proportion of exfoliating particles having a diameter of 150 to 450 µm is 60 to 75% by weight and wherein
- the preparations according to the invention comprise at least one preservative from the group comprising benzoic acid, a salt of benzoic acid, salicylic acid and a salt of salicylic acid or wherein
the preparations according to the invention comprise at least one preservative from the group comprising methylparaben and ethylparaben.

2. Preparation according to Claim 1, **characterized in that** the exfoliating particles consist only of a waxy substance, preferably hydrogenated castor oil.

3. Preparation according to Claim 1, **characterized in that** an exfoliating particle consists of two or more waxy substances.

4. Preparation according to Claim 2 and/or 3, **characterized in that** the exfoliating particles are mixtures of exfoliating particles, wherein the exfoliating particles in each case consist only of one waxy substance or of two or more waxy substances.

5. Preparation according to at least one of the preceding claims, **characterized in that** further exfoliating particles consisting of at least one substance selected from the group comprising hydrated silica, polylactic acid and microcrystalline cellulose are present.

6. Preparation according to at least one of the preceding claims, **characterized in that** the ratio by weight of the spherical exfoliating particles and consisting of at least one waxy substance to the exfoliating particles consisting of at least one substance selected from the group comprising hydrated silica, polylactic acid and microcrystalline cellulose is from 10:1 to 1:10, preferably from 2:3 to 3:2.

7. Preparation according to any of the preceding claims, **characterized in that** the total content of exfoliating particles is 0.05 to 15% by weight, preferably 0.075 to 10% by weight, particularly preferably 0.1 to 7% by weight, based on the total weight of the preparation.

8. Preparation according to at least one of the preceding claims, **characterized in that** the predominant proportion of exfoliating particles a particle size diameter of ≤ 600 µm, wherein it is preferred that the proportion of exfoliating particles having a diameter of 250 to 420 µm is 80% by weight or more.

9. Preparation according to at least one of the preceding claims, **characterized in that** the amphoteric surfactants are alkyl betaines and/or alkylamidopropyl betaines, wherein the alkyl betaines and/or alkylamidopropyl betaines comprise alkyl radicals having a chain length of 8 to 16 carbon atoms, particularly alkyl radicals having a chain length of 10 to 14 carbon atoms and very particularly alkyl radicals having a chain length of 11 to 13 carbon atoms.

10. Preparation according to at least one of the preceding claims, **characterized in that** the alkyl betaines and/or alkylamidopropyl betaines comprise alkyl groups derived from the processing of coconut oil to surfactants.

11. Preparation according to Claim 10, **characterized in that** the alkyl betaines and/or alkylamidopropyl betaines are coco-betaine and/or cocamidopropyl betaine.

12. Preparation according to at least one of the preceding claims, **characterized in that** the non-ionic surfactants ethoxylated glycerol esters.

13. Preparation according to at least one of the preceding claims, **characterized in that** the ethoxylated glycerol esters comprise alkyl radicals having a chain length of 8 to 16 carbon atoms, particularly 10 to 14 carbon atoms, very particularly 11 to 13 carbon atoms.

14. Preparation according to at least one of the preceding claims, **characterized in that** the ethoxylated glycerol esters are PEG-7 glyceryl cocoate.

15. Preparation according to at least one of the preceding claims, **characterized in that** the preparations are free from haloorganic preservatives.

16. Preparation according to at least one of the preceding claims, **characterized in that** the preparations are free from paraben-containing preservatives comprising an alkyl side chain of more than 3 carbon atoms.

## Revendications

1. Préparations nettoyantes de la peau, contenant des tensioactifs et des particules d'exfoliation, les particules d'exfoliation étant sphériques et constituées par au moins une substance de type cire et les préparations étant exemptes de conservateurs dissociant du formaldéhyde, de 2-bromo-2-nitropropane-1,3-diol et de butylparabène,
- les tensioactifs étant choisis dans le groupe des tensioactifs anioniques, amphotères et non ioniques et les tensioactifs étant présents en une teneur de 5 à 18% en poids, de préférence de 10 à 15 % en poids, en particulier de préférence de 10 à 13 % en poids, par rapport au poids total de la préparation,
- les tensioactifs anioniques étant des sels de métal alcalin et/ou d'ammonium d'alkyléthersulfates, les alkyléthersulfates présentant des radicaux alkyle d'une longueur de chaîne de 8 à 16 atomes de carbone, en particulier des radicaux alkyle d'une longueur de chaîne de 10 à 14 atomes de carbone et tout particulièrement des radicaux alkyle d'une longueur de chaîne de 11 à 13 atomes de carbone,
- les substances de type cire étant choisies dans le groupe formé par la cire de carnauba, la cire de candelilla, la cire de tournesol, l'huile de ricin hydrogénée, la cire de riz, l'huile de tournesol hydrogénée, l'huile de soja hydrogénée, la paraffine, où la paraffine dure, la cire microcristalline et la vaseline sont incluses, et le stéarate de stéaryle et
- la proportion principale des particules d'exfoliation présentant une grosseur de particule d'un diamètre □ 850 µm, la proportion de particules d'exfoliation présentant un diamètre de 150 à 450 µm représentant de préférence 60 à 75 % en poids et
- les préparations selon l'invention contenant au moins un conservateur du groupe formé par l'acide benzoïque, un sel de l'acide benzoïque, l'acide salicylique et un sel de l'acide salicylique ou
- les préparations selon l'invention contenant au moins un conservateur du groupe formé par le méthylparabène et l'éthylparabène.

2. Préparation selon la revendication 1, **caractérisée en ce que** les particules d'exfoliation ne sont constituées que d'une seule substance de type cire, de préférence d'huile de ricin hydrogénée.

3. Préparation selon la revendication 1, **caractérisée en ce qu'**une particule d'exfoliation est constituée par plusieurs substances de type cire.

4. Préparation selon la revendication 2 et/ou 3, **caractérisée en ce que** les particules d'exfoliation sont des mélanges de particules d'exfoliation, les particules d'exfoliation n'étant à chaque fois constituées que par une seule substance de type cire ou par plusieurs substances de type cire.

5. Préparation selon au moins l'une quelconque des revendications précédentes, **caractérisée en ce que** les particules d'exfoliation supplémentaires constituées par au moins une substance choisie dans le groupe formé par la silice hydratée, le poly(acide lactique) et la cellulose microcristalline sont contenues.

6. Préparation selon au moins l'une quelconque des revendications précédentes, **caractérisée en ce que** le rapport pondéral des particules d'exfoliation sphériques et constituées par au moins une substance de type cire aux particules d'exfoliation constituées par au moins une substance choisie dans le groupe formé par la silice hydratée, le poly(acide lactique) et la cellulose microcristalline est de 10:1 à 1:10, de préférence de 2:3 à 3:2.

7. Préparation selon l'une quelconque des revendications précédentes, **caractérisée en ce que** la teneur totale en particules d'exfoliation représente 0,05 à 15 % en poids, de préférence 0,075 à 10 % en poids, en particulier de préférence de 0,1 à 7 % en poids par rapport au poids total de la préparation.

8. Préparation selon au moins l'une quelconque des revendications précédentes, **caractérisée en ce que** la proportion principale des particules d'exfoliation une grosseur de particule d'un diamètre ≤ 600 µm, la proportion de particules d'exfoliation présentant un diamètre de 250 à 420 µm représentant de préférence 80 % en poids ou plus.

9. Préparation selon au moins l'une quelconque des revendications précédentes, **caractérisée en ce que** les tensioactifs amphotères sont des alkylbétaïnes et/ou des alkylamidopropylbétaïnes, les alkylbétaïnes et/ou les alkylamidopropylbétaïnes présentant des radicaux alkyle d'une longueur de chaîne de 8 à 16 atomes de carbone, en particulier des radicaux alkyle d'une longueur de chaîne de 10 à 14 atomes de carbone et tout particulièrement des radicaux alkyle d'une longueur de chaîne de 11 à 13 atomes de carbone.

10. Préparation selon au moins l'une quelconque des revendications précédentes, **caractérisée en ce que** les alkylbétaïnes et/ou les alkylamidopropylbétaïnes présentent des groupes alkyle qui proviennent de la transformation d'huile de coco en tensioactifs.

11. Préparation selon la revendication 10, **caractérisée en ce que** les alkylbétaïnes et/ou les alkylamidopropylbétaïnes sont des bétaïnes de coco et/ou des cocoamidopropylbétaïnes.

12. Préparation selon au moins l'une quelconque des revendications précédentes, **caractérisée en ce que** les tensioactifs non ioniques des esters de glycérol éthoxylés.

13. Préparation selon au moins l'une quelconque des revendications précédentes, **caractérisée en ce que** les esters de glycérol éthoxylés présentent des radicaux alkyle d'une longueur de chaîne de 8 à 16 atomes de carbone, en particulier de 10 à 14 atomes de carbone et tout particulièrement de 11 à 13 atomes de carbone.

14. Préparation selon au moins l'une quelconque des revendications précédentes, **caractérisée en ce que** les esters de glycérol éthoxylés sont des cocoates de PEG-7-glycéryle.

15. Préparation selon au moins l'une quelconque des revendications précédentes, **caractérisée en ce que** les préparations sont exemptes de conservateurs halogéno-organiques.

16. Préparation selon au moins l'une quelconque des revendications précédentes, **caractérisée en ce que** les préparations sont exemptes de conservateurs contenant des parabènes présentant une chaîne alkyle latérale de plus de 3 atomes de carbone.
